# EUROPEAN PATENT SPECIFICATION

(11) **EP 0 361 839 B1**
(45) Date of publication and mention of the grant of the patent: **15.12.1993**
(21) Application number: 89309748.5
(22) Date of filing: 26.09.1989
(51) Int. Cl.: B01D 3/36

(54) **Dehydration process using azeotropic distillation**
Verfahren zum Entwässern durch azeotrope Destillation
Procédé de déshydratation par distillation azéotrope

(30) Priority: 26.09.1988 HU 500688
(43) Date of publication of application: 04.04.1990
(73) Proprietor: RICHTER GEDEON VEGYESZETI GYAR R.T., H-1475 Budapest (HU)
(72) Inventor: Greiner, Istvan, H-1119 Budapest (HU); Stefko, Bela, H-1117 Budapest (HU); Szilbereky, Jeno, H-1122 Budapest (HU); Thaler, Gyorgy, H-1015 Budapest (HU)
(74) Representative: Pett, Christopher Phineas

(56) References cited:
- EP-A- 0 186 816
- DE-A- 2 126 505
- DE-A- 3 426 080
- DE-C- 851 050
- DE-C- 893 195

## Description

The present invention relates to a dehydration process. More particularly it relates to a process for the vigorous dehydration of substances or mixtures, primarily condensation reaction mixtures, using continuous azeotropic distillation with an organic solvent which forms with water a practically immiscible azeotropic mixture of minimal boiling point.

In many areas of the chemical industry there is a need for dehydration processes. Amongst the most important of these is the continuous dehydration of reaction mixtures originating from reactions which involve the liberation of water. Examples of such reactions, known as condensation reactions, are direct esterification (the reaction of an organic acid or acid anhydride and an alcohol), direct amidation (the reaction of an organic acid or acid anhydride and an amine) and formation of an acetal or ketal (the reaction of an aldehyde or ketone and an alcohol). The continuous removal of water from the reaction mixture during reactions of this kind shift the position of equilibrium in the direction of product formation, thus driving the reaction further to completion.

On an industrial scale, water is continuously removed from the reaction mixture by extraction or by azeotropic distillation. The essence of an extraction is to add an organic solvent which is practically immiscible with water to the reaction mixture, so that the water formed during the reaction separates from the reaction medium. This is reported in the literature, in, for example J. Am. Chem. Soc., 70, 3135 (1948) and J. Pharm. Pharmacol. 2, 229 (1950). For the removal of water by azeotropic distillation, an organic solvent, which forms a practically immiscible azeotropic mixture of minimal boiling point with water, is added to the reaction mixture, and the vapours formed during boiling of the reaction mixture are condensed away from the boiling reaction mixture of the two phases, and the organic phase containing a little water is recirculated back to the reaction mixture.

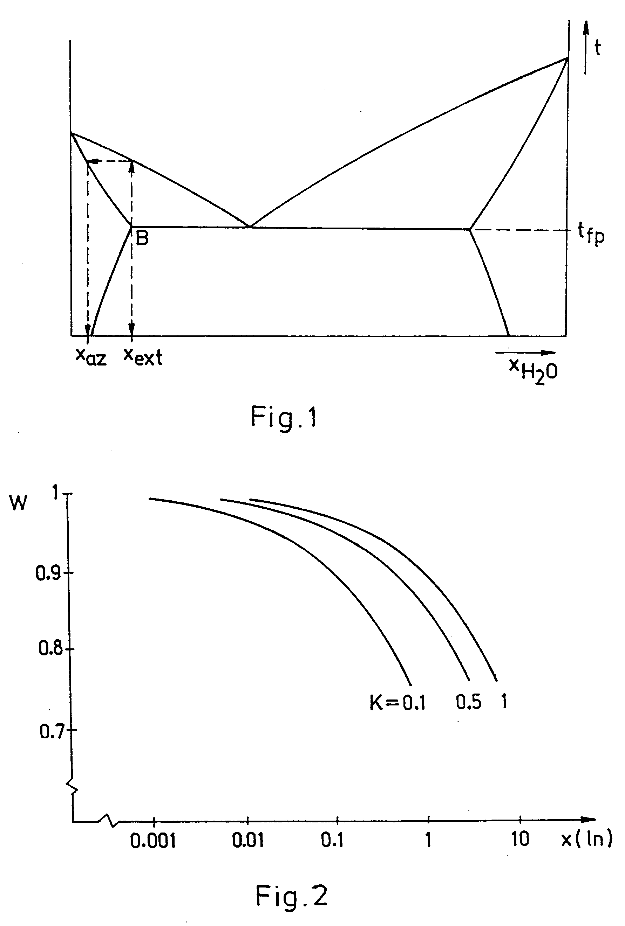

The minimal water concentration theoretically obtainable in a reaction mixture by such extraction and azeotropic distillation methods is shown in Figure 1 of the accompanying drawings. This shows a phase-diagram of a two-component system of water and an organic solvent substantially immiscible with water, but which forms with water an azeotropic mixture of minimal boiling point.

In Figure 1, xₑₓₜ and x_{az} represent the water concentrations obtainable by the extraction method and azeotropic distillation described above respectively.

During water removal carried out by the extraction method, the water content of the reaction mixture cannot be reduced below the xₑₓₜ water concentration value, because this water concentration value corresponds to the water concentration of the organic phase being in equilibrium with the water phase at the boiling point (point B). In the case of a water content lower than xₑₓₜ, the organic phase and the water do not separate into two phases.

During water removal carried out by azeotropic distillation, the water content of the reaction mixture cannot be reduced below the x_{az} value shown, because by condensing the distillate of a reaction mixture of this composition, point B is reached in Figure 1. At B, the liquid state distillate does not separate to two phases, and this means that no more water can be removed from the condensing vapour, and thus, from the total amount of water in the reaction mixture.

A batch-wise azeotropic distillation method for the removal of water is described in the German patent specification No. 3,335,312. According to this method, organic solvents freely miscible with water are used as azeotropic partners. The disadvantage of the method is that the regeneration of these solvents is difficult and a rectification column is needed for the dehydration of the solvent. This makes the procedure complicated and expensive.

Water removal carried out by azeotropic distillation has been carried out using organic solvents both lighter and heavier than water. From among the solvents lighter than water, benzene, toluene and diisopropyl ether may be used advantageously [c.f.: Deák, Gy.: "Szerves vegyipar alapfolyamatok kézikönyve" (Handbook of the basic processes of the organic chemistry - in Hungarian), 455 (1978), and DE-A-29,17,087], but many examples are known from the literature for the application of organic solvents heavier than water, for example carbon tetrachloride or 1,2-dichloroethane [Org. Synt. Coll. Vol. I. 261 (1955) and the US patent specification No. 2,010,436].

Water removal carried out by azeotropic distillation is still currently used for dehydration of substances and mixtures [Kirk-Othmer: Encyclopedia of Chemical Technology, Azeotrope Distillation; III. Edition, Wiley-Interscience (1976)].

The disadvantage of azeotropic distillation methods described above is that not all the water formed in the reaction can be removed by this method; the extent of removal is insufficient to complete the reaction. However, compared to the azeotropic distillation method, the extraction method is even more disadvantageous, because the extent of water removal by this method is lower than with known azeotropic distillation methods.

The most frequent area of application for water removal, carried out by azeotropic distillation, takes place in direct esterification reactions. In these reactions and in the case of direct acetal and ketal formation, there is one more disadvantage. Often in such reactions, a 3-5-fold excess of the alcohol reactant having a short carbon chain, mostly 1-4 carbon atoms, is used. The boiling point of such alcohols is close to the boiling point of the solvent used to form the azeotropic mixture of minimal boiling point with the water. Because of the closeness of the boiling points, a significant amount of the alcohol is distilled with the azeotropic mixture, and this results in significant dissolution between the separating phases in the condensate.

In many cases there is a need for a higher degree of dehydration than that attainable by using known processes. This is the case mostly with equilibrium reactions in which the value of the equilibrium constant is around unity, or the equilibrium is shifted towards hydrolysis, that is, contrary to the desired reaction. This is the situation for example in the esterification of pyruvic acid, oxalic acid, ethylmalonic acid or phthalic acid, and in the amidation of formic acid. In these cases, dehydration is carried out by recirculating the refluxing organic phase, removed from the reaction mixture by distillation, through a solid desiccant, e.g. CaCl₂, P₂O₅, K₂CO₃, before its return to the reaction mixture [Org. Synth. Coll. Vol. I., 261 (1955)]. According to the method described in J. Org. Chem. 48, 3106 (1983) the reaction mixture is passed through a column filled with a solid desiccant and the low water concentration necessary for the favourable chemical transformation is produced this way.

On an industrial scale, the application of solid desiccants had the disadvantage that a huge amount of desiccant must be used, and the subsequent regeneration of the desiccant is difficult. These factors make the actual use of such a reaction method difficult and expensive.

We have therefore sought to develop an improved process for the continuous removal of water from substances or mixtures including condensation reaction mixtures. In the case of condensation reactions a further aim is to shift the reaction equilibrium in the direction of product formation, and so increase the yield of the product. This we have been able to achieve.

The invention is based on the recognition that in the case of dehydration carried out by azeotropic distillation, the water content of substances, and mixtures, including condensation reaction mixtures, can be reduced below the water content normally attainable by know azeotropic distillation methods if the condensate with the given water content is cooled to a temperature at which the practically immiscible organic solvent, which forms with water the azeotropic mixture of minimal boiling point, becomes supersaturated in respect of water. As a result, further water separation occurs, and an organic phase with a lower water content can be recycled back into the distillation vessel.

This recognition of supersaturation is significant especially in the range of water concentration marked in Figure 1 by point B and below. With the known azeotropic distillation methods, wherein the separating phases of the distillate are not cooled, it is theoretically impossible to reach a lower water concentration than the one marked by x_{az} in the figure.

For dehydration of reaction mixtures resulting from direct esterification, acetal and ketal formation, we have also recognized that if a solvent forming a practically immiscible azeotropic mixture of minimal boiling point with water and having a higher density than water, is used, the amount of alcohol to be used as a reagent may be reduced close to the theoretical amount necessary. The organic solvent continuously perforates the alcohol dissolved in the aqueous phase during the reaction as it is heated, and thus the alcohol is recycled with the organic phase back into the reaction medium. The reciprocal increase in solubility between the water and organic solvent, caused by the alcohol, disappears or at least decreases the less alcohol is used in the reaction.

Thus, in one aspect, the invention provides a process for the dehydration of substances or mixtures primarily condensation reaction mixtures, by continuous azeotropic distillation with an organic solvent which forms with water a practically immiscible azeotropic mixture of minimal boiling point wherein the condensing distillate is cooled to at least a temperature at which the condensate with a given water content, or the organic phase of this condensate, is supersaturated with water, and the resulting organic phase with reduced water content is recycled back into the distillation vessel.

In the process according to the present invention the condensing distillate is suitably cooled to a temperature at which, of the now separate phases, that recycled back into the reaction mixture is still in a liquid state, that is, above the melting point of the organic phase saturated with water.

The process according to the present invention can be advantageously realized in the case of condensation reactions which go to equilibrium, in which the increased water removal results in an increase of the yield. Especially good results can be reached for those condensation reactions in which the value of the equilibrium constant (K) is unity or less than unity. Figure 2 of the accompanying drawings shows that the theoretical yield (w) concerning the product (C) of the condensation reaction characterized by the equation

$\text{A + B = C + H₂O}$

Even in cases of low water content values (x is the water content of the mixture in mol%), w is significantly altered. For example, if the water content in the reaction mixture is reduced, using the process according to the present invention, to 0.02 mol%, as opposed to the 0.2 mol% reached by the known procedures, then this results in a theoretical yield increase of 4 to 10% in the case of reactions having an equilibrium constant of unity or less than unity.

In those condensation reactions in which one of the reagents is a short-chain alcohol, known procedures use a 3 to 5-fold excess of the alcohol in order to shift the reaction into the direction of product formation. From the point of view of dehydration, the excess alcohol has the disadvantage mentioned above, that is, it increases the solubility of water in the solvent used for the azeotropic distillation. Using the process according to the present invention it is possible to reduce the amount of alcohol required for the reaction to close to the theoretical amount necessary (about 5-10 mol% excess), and provide a simultaneous increase in product yield.

According to a further feature of the process of the present invention, the solvent which is practically immiscible with water can be both of higher density than water, preferably 1,2-dichloroethane, chloroform or carbon tetrachloride, or of lower density than water, preferably benzene or toluene.

The process according to the present invention is illustrated by the following non-limiting examples.

The literature sources of the yield data reported in the examples for comparison, may be found in the following;
(1) J. Chem. Soc., 532 (1948) (extractive method, using benzene)
(2) J. Am. Chem. Soc., 70, 3135 (1948) (extractive method)
(3) Annalen 571, 53, (1951) (extractive method), the reproduction of the procedure of (2)
(4) Org. Synth. Coll. Vol. III., 610 (1955) (azeotropic method using benzene)
(5) J. Am. Chem. Soc., 66, 1657 (1944) (azeotropic method using carbon tetrachloride)
(6) Org. Synth. Col. Vol. I., 261 (1955) (azeotropic method using carbon-tetrachloride, drying with K₂CO₃)
(7) J. Pharm. Pharmacol., 2,229 (1950) (extractive method)
(8) US patent No. 2,010,425 from 1935 (azeotropic method using 1,2-dichloroethane)
(9) DE-A 29,17,087 from 1979 (azeotropic method using diisopropyl ether)
(10) Bulletin de la Societe Chimique de Belgique, 28, 339 (1922) (extractive method)

### Example 1

66 g of ethylmalonic acid are dissolved in a mixture of 200 ml of benzene and 64 g of methanol. 0.5 ml of concentrated sulphuric acid are added to the reaction mixture. It is refluxed for 10 hours through a water separating unit. The distillate collected in the unit is cooled to 6°C. After the reaction the reaction mixture is quickly neutralized by adding 34 ml of a 5% by weight icy aqueous sodium hydrogencarbonate solution. The organic phase is dried and distilled fractionally.

54 g (67.5%) of dimethyl ethylmalonate are obtained, boiling point is 98°C at a pressure of 3300 Pa.

Yield in the literature: 50% (1).

In Table I examples are summarized which demonstrate the yield increase obtainable by dehydration in cases of esterification reactions. Table I contains the parameters of the processes, and the yield data of esterifications measured and known from the literature, respectively.

The esterification processes for Table 1 are carried out as follows:
1 mole of a carboxylic acid or anhydride is dissolved or suspended in a mixture of 1,2-dichloroethane and, calculated on the amount of the acid or anhydride, an alcohol in an excess or 10 mole%. If necessary, catalyst is added to the reaction mixture, and the mixture is refluxed for 12 hours through a cooled water separating unit. The reaction mixture is quickly neutralized with icy 5% by weight aqueous sodium hydrogencarbonate solution, added in an amount corresponding to the theoretical amount necessary. The organic phase is dried and fractionally distilled.

| No. of the example | Product | Catalyst | Cooling temperature | Yield | |
|---|---|---|---|---|---|
| | | | | measured | literature |
| 2 | dimethyl ethylmalonate | cc.sulphuric acid | 10 °C | 85-88% | 50% (1) |
| 3 | methyl pyruvate | PTSA | 5 °C | 78-82% | 73% (2) |
| | | | | | 59% (3) |
| | | | | | 65-71% (4) |
| 4 | ethyl pyruvate | PTSA | 5 °C | 77-82% | 59% (5) |
| 5 | diethyl oxalate | - | 10 °C | 93-95% | 80-85% (6) |
| | | | | | 48-51% (7) |
| 6 | dibutyl phthalate | cc.sulphuric acid | 10 °C | 98-99% | 96% (8) |
| PTSA = p-toluene sulphonic acid | | | | | |

In Examples 2, 3, 4 and 5 the corresponding carboxylic acid is the starting material. The starting material of the product according to the Example 5 is a dihydrated compound. The starting material in Example 6 is phthalic acid anhydride.

### Example 7

A mixture of 30 ml of formic acid, 31 g of aniline and 120 ml of 1,2-dichloroethane is boiled for 12 hours using a water separating unit. The distillate collected in the unit is continuously cooled to 10°C. The product is isolated by fractional distillation from the reaction mixture previously neutralized with icy, 5% by weight aqueous sodium hydrogencarbonate solution.

39.2 g of formanilide are obtained with a yield of 97%. The boiling point of the product is 124°C/200 Pa, the melting point is 49-50°C.

Literature data: yield 91.7%, boiling point 146°C/460 Pa, melting point 47-48.5°C (9).

### Example 8

A mixture of 36 g (0.5 moles) of butyric aldehyde, 81.4 g (1.1 moles) of n-butyl alcohol, 0.2 g of p-toluene sulphonic acid and 250 ml of 1,2-dichloroethane is refluxed for 14 hours through a water separating unit. The distillate collected in the unit is constantly cooled below 10°C. The reaction mixture is extracted with a 5% by weight aqueous sodium hydrogencarbonate solution added in excess, and the organic phase is dried and fractionally distilled.

74.7 g of dibutyl butanal are obtained with a yield of 74%. The boiling point of the product is 106-112°C/-2500 Pa.

Literature data: yield 13%, boiling point 100-106°C/-2400 Pa (10).

We have found the following advantages of the process of the present invention:
a) the water content of the organic solvent forming with water a practically immiscible azeotropic mixture of minimal boiling point in the condensate is lower, and because of this the water content in the distillion vessel may be significantly reduced, compared to known methods;
b) the process can be used on an industrial scale, and the extra required apparatus is negligible;
c) water removal can be carried out in an environmental protective way in the case of condensation reactions;
d) because of the lower water content, 5-30 percent yield increases are obtainable , calculated on the final product;
e) because of the lower water content of the refluxing organic phase, the water concentration is lower in the reaction mixture than with the earlier processes whilst equilibrium is being reached, and as a result of this the rate of hydrolysis will be lower during the reaction, and the reaction time will be significantly shorter;
f) in many cases significant energy saving can be reached with the shortening of reaction time;
g) the processing of the reaction mixture and the purification of the final product are significantly easier since as a result of the further completion of the reaction, the amount of unused reactants left is significantly less.

## Claims

1. A process for the dehydration of substances or mixtures carried out by continuous azeotropic distillation with an organic solvent which forms with water a practically immiscible azeotropic mixture of minimal boiling point wherein the condensing distillate is cooled to at least a temperature at which the condensate with a given water content or the organic phase of the condensate is supersaturated with water, thus enabling further water separation, and the resulting organic phase of reduced water content is recycled back into the distillation vessel.

2. A process as claimed in Claim 1 wherein the condensing distillate is cooled at most to the melting point of the organic solvent saturated with water.

3. A process as claimed in Claim 1 or Claim 2, wherein during the continuous dehydration of direct esterification, direct acetal formation or direct ketal formation reaction mixtures, a short-chain alcohol used in the reaction mixture as a starting material is used in an excess of at most 10 mole% and the organic solvent used to form with water a practically immiscible azeotropic mixture of minimal boiling point has a density higher than water.

4. A process as claimed in any one of Claims 1 to 3 wherein the organic solvent is an halogenated aliphatic hydrocarbon.

5. A process as claimed in claim 4 wherein the halogenated aliphatic hydrocarbon is 1,2-dichloroethane, chloroform or carbon tetrachloride.

6. A process as claimed in claim 1 or claim 2 wherein the organic solvent is an aromatic hydrocarbon.

7. A process as claimed in claim 6 wherein the aromatic hydrocarbon is benzene or toluene.

## Patentansprüche

1. Verfahren zur Dehydratisierung von Substanzen oder Mischungen, durchgeführt durch kontinuierliche azeotrope Destillation mit einem organischen Lösungsmittel, das mit Wasser eine praktisch unmischbare azeotrope Mischung mit minimalem Siedepunkt bildet, wobei das Kondensationsdestillat auf zumindest eine Temperatur gekühlt wird, bei der das Kondensat mit einem gegebenen Wassergehalt oder die organische Phase des Kondensates mit Wasser übersättigt wird, wodurch eine weitere Wassertrennung ermöglicht wird, und wobei die resultierende organische Phase mit vermindertem Wassergehalt zu dem Destillationskessel zurückgeführt wird.

2. Verfahren gemäß Anspruch 1, dadurch **gekennzeichnet,** daß das Kondensationsdestillat maximal auf den Schmelzpunkt des organischen Lösungsmittels, das mit Wasser gesättigt ist, gekühlt wird.

3. Verfahren nach Anspruch 1 oder nach Anspruch 2, dadurch **gekennzeichnet,** daß während der kontinuierlichen Dehydratisierung von direkten Veresterungs-, direkten Azetalbildungs- oder direkten Ketalbindungsreaktionsmischungen ein bei der Reaktionsmischung als ein Ausgangsmaterial verwendeter kurzkettiger Alkohol in einem Überschuß von maximal 10 Mol-% verwendet wird, und daß das organische Lösungsmittel, das zur Bildung einer praktisch unmischbaren azeotropen Mischung mit minimalem Siedepunkt mit Wasser verwendet wird, eine höhere Dichte als Wasser aufweist.

4. Verfahren nach einem der Ansprüche 1 bis 3, dadurch **gekennzeichnet,** daß das organische Lösungsmittel ein halogenierter aliphatischer Kohlenwasserstoff ist.

5. Verfahren nach Anspruch 4, dadurch **gekennzeichnet,** daß der halogenierte aliphatische Kohlenwasserstoff 1,2-Dichlorethan, Chloroform oder Tetrachlorkohlenstoff ist.

6. Verfahren nach Anspruch 1 oder 2, dadurch **gekennzeichnet,** daß das organische Lösungsmittel ein aromatischer Kohlenwasserstoff ist.

7. Verfahren nach Anspruch 6, dadurch **gekennzeichnet,** daß der aromatische Kohlenwasserstoff Benzol oder Toluol ist.

## Revendications

1. Procédé de déshydratation de substances ou de mélanges, effectué par distillation azéotrope continue avec un solvant organique qui forme un mélange azéotrope pratiquement non miscible avec l'eau, ayant un point d'ébullition minimal, dans lequel le condensat de distillation est refroidi à une température minimale à laquelle le condensat ayant une teneur définie en eau ou la phase organique du condensat est sursaturé d'eau, et la phase organique résultante à teneur réduite en eau est recyclée dans le récipient de distillation, ce qui permet une séparation complémentaire de l'eau.

2. Procédé selon la revendication 1, dans lequel le distillat de condensation est refroidi au plus jusqu'au point de fusion du solvant organique saturé d'eau.

3. Procédé selon la revendication 1 ou 2, dans lequel, pendant la déshydratation continue de mélanges de réaction à estérification directe, à formation directe d'acétal ou à formation directe de cétal, un alcool à chaîne courte, utilisé comme produit de départ dans le mélange de réaction, est utilisé en un excès d'au plus 10 % en moles, et le solvant organique utilisé pour former un mélange azéotrope pratiquement non miscible avec l'eau, ayant un point d'ébullition minimal, a une densité supérieure à l'eau.

4. Procédé selon l'une quelconque des revendications 1 à 3, dans lequel le solvant organique est un hydrocarbure aliphatique halogéné.

5. Procédé selon la revendication 4, dans lequel l'hydrocarbure aliphatique halogéné est le 1,2-dichloroéthane, le chloroforme ou le tétrachlorure de carbone.

6. Procédé selon la revendication 1 ou 2, dans lequel le solvant organique est un hydrocarbure aromatique.

7. Procédé selon la revendication 6, dans lequel l'hydrocarbure aromatique est le benzène ou le toluène.
